(19) Europäisches Patentamt

European Patent Office

Office européen des brevets

(11) Veröffentlichungsnummer: **0 301 058**
**B1**

(12) **EUROPÄISCHE PATENTSCHRIFT**

(45) Veröffentlichungstag der Patentschrift: **12.09.90**

(21) Anmeldenummer: **88901536.8**

(22) Anmeldetag: **25.02.88**

(86) Internationale Anmeldenummer:
**PCT/CH88/00045**

(87) Internationale Veröffentlichungsnummer:
**WO 88/06534 07.09.88 Gazette 88/20**

(51) Int. Cl.⁵: **B 43 K 29/00**

(54) **KUGELSCHREIBER MIT KONDOM.**

(30) Priorität: **25.02.87 CH 705/87**

(43) Veröffentlichungstag der Anmeldung:
**01.02.89 Patentblatt 89/05**

(45) Bekanntmachung des Hinweises auf die
Patenterteilung:
**12.09.90 Patentblatt 90/37**

(84) Benannte Vertragsstaaten:
**AT BE CH DE FR GB IT LI LU NL SE**

(56) Entgegenhaltungen:
**DE-A- 417 248**

(73) Patentinhaber: **WEY, Remo Curt**
**31, rue près Guetins**
**CH-2520 La Neuveville (CH)**

(72) Erfinder: **WEY, Remo Curt**
**31, rue près Guetins**
**CH-2520 La Neuveville (CH)**

(74) Vertreter: **Feldmann, Clarence Paul et al**
**c/o Patentanwaltsbüro FELDMANN AG Postfach**
**Kanalstrasse 17**
**CH-8152 Glattbrugg (CH)**

EP 0 301 058 B1

## Beschreibung

Da die Verwendung von Kondomen seit der Ausbreitung der Immunschwäche AIDS zur Vermeidung von Ansteckung wieder einen starken Aufschwung erfahren hat, besteht auch das Bedürfnis, dieselben gut geschützt von Beschädigung und diskret mitführen zu können.

Aus der DE—C—417248 ist ein Schreibstift bekannt geworden, der in eine Hülse übergeht, in der Toilettengegenstände untergebracht sind, so dass diese diskret mitgeführt werden können. Es handelt sich in diesem Fall um eine kleine Puderquaste und einen Lippenstift. Sowohl der Lippenstift, wie die Puderquaste haben einen eingenen Griff mit dem sie bequem aus der Hülse herausgezogen werden können.

Ist dagegen ein zusammengefaltetes Kondom auf ähnliche Weise in einer möglichst engen Hülse eines Kugelschreibers untergebracht, lässt es sich nur schwer daraus entnehmen. Würde man eine Hülse mit grösserem Durchmesser verwenden, wäre sofort erkennbar, dass es sich nicht um einen normalen Kugelschreiber handelt und ginge die gewünschte Diskretion verloren.

Die Erfindung hat sich daher die Aufgabe gestellt, einen Kugelschreiber mit Kondom zu schaffen, wobei sich das Kondom oder die darin untergebrachten Kondome bequem entnehmen lässt beziehungsweise lassen. Gestaltet man den Kugelschreiber so, dass die Hülse aus lösbar mit dem Kopfteil und untereinander verbundenen Hülsenteilen besteht, die je ein Kondom enthalten, so dienen diese Hülsenteile auch als Verpackung während des Verkaufs und vermeiden eine Verletzung bei der Nachfüllung durch den Benützer.

In den beigefügten Zeichnungen sind Ausführungsbeispiele des Erfindungsgegenstandes dargestellt. Es Zeigt:

Figur 1 einen Kugelschreiber mit einer mehrteiligen Hülse;

Figur 2 denselben Kugelschreiber in auseinander Genommenem Zustand;

Figur 3 einen Kugelschreiber mit einteiliger Hülse und Schieber.

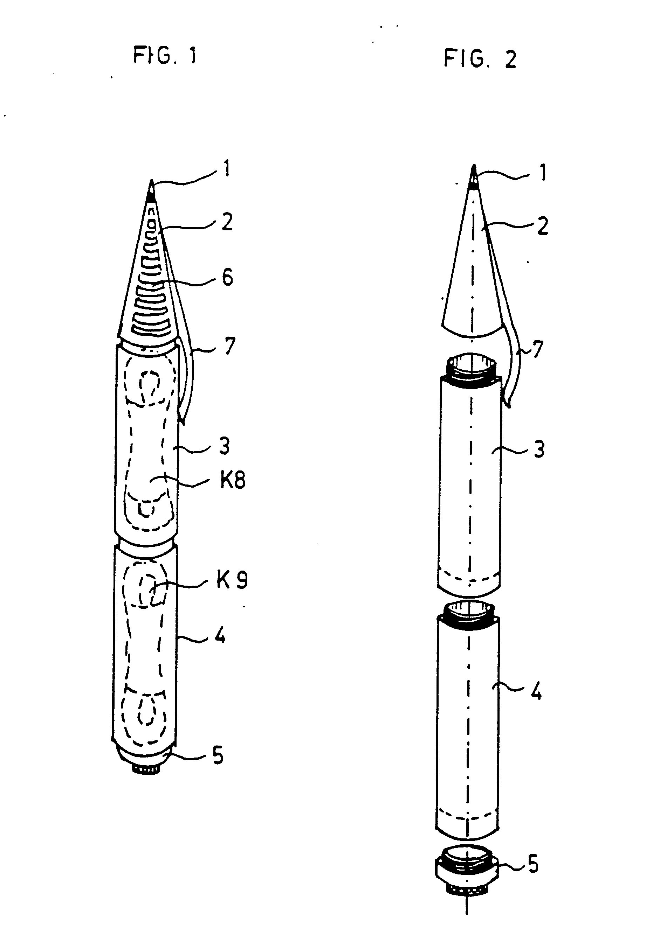

Der in Figur 1 dargestellte Kugelschreiber besitzt einen Schreibkopf mit einer Schreibspitze 1, eine mit einem Klip 7 versehene konische Hülse 2 in die ein die Spitze 1 tragender konischer Schreibflüssigkeitsbehälter 6 eingesetzt ist. Am Kopf ist eine erste ein Kondom K/8 enthaltende Teilhülse 3 angesetzt, vorzugsweise angeschraubt und an dieser wieder in gleicher Weise wieder eine Hülse 4 mit einem Kondom K/9. Diese zweite Teilhülse 4 ist durch eine aufgeschraubte oder aufgesteckte Kappe 5 verschlossen. Durch den zerlegbaren Aufbau der Hülse können die Kondome bequem einzeln entnommen werden. Die Teilhülsen können beispielsweise stirnseitig mit einem Aussengewinde und rückseitig mit einem Innengewinde vesehen sein, ohne dass dabei andere Verbindungsmöglichkeiten ausgeschlossen seien. Der Aufbau erlaubt es, die Kondome K8, K9 somit in solchen Teilhülsen als Einwegverpackung auf den Markt zu bringen.

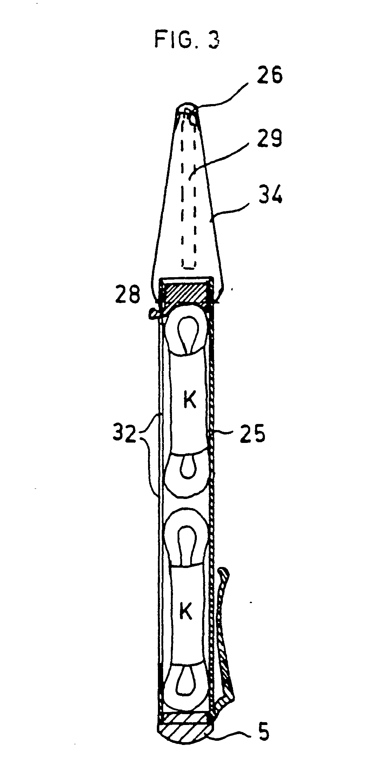

Bei der Ausführungsform nach Figur 3 ist die Schreibspitze mit 26 und die Mine mit 29 bezeichnet. Der Schreibkopf ist wieder fest mit der Hülse 25 verbunden, in welche zwei Kondome K untergebracht sind. Die Hülse 25 weist einen Längsschlitz 32 auf, durch den ein Griffteil eines Schiebers 28 herausragt. Es ist leicht ersichtlich, dass mit Hilfe dieses Schiebers 28 die Kondome bequem aus der Hülse 25 herausgeschoben werden können. Die Abdeckhülse für den Schreibkopf ist hier mit 34 bezeichnet.

## Patentansprüche

1. Kugelschreiber mit Kondom, dadurch gekennzeichnet, dass er einen Kopfteil (1, 2, 34) mit Schreibspitz (1, 26) und darin angeordneter Mine (6, 29) aufweist, dass der Kopfteil in eine endseitig (5) verschliessbare ein- oder mehrteilige Hülse (3, 4, 25) übergeht, in der mindestens ein Kondon (K8, K9, K) untergebracht ist, und dass diese Hülse mit Mitteln (28, 32) versehen ist, die ein Ausbringen der Kondome (K8, K9, K) aus der Hülse erleichtern.

2. Kugelschreiber nach Anspruch 1, dadurch gekennzeichnet, dass die Hülse aus lösbar mit dem Kopfteil und untereinander verbundenen Hülsenteilen (3, 4) besteht, die je ein Kondom (K8, K9) enthalten.

3. Kugelschreiber nach Anspruch 2, dadurch gekennzeichnet, dass die Hülsenteile (3, 4) untereinander und mit dem Kopfteil (1, 2) verschraubt sind.

4. Kugelschreiber nach Anspruch 1, dadurch gekennzeichnet, dass in der Hülse ein Schieber (28) angebracht ist, der mittels eines durch einen Längsschlitz (32) nach aussen ragenden Griffteil verschiebbar ist.

## Revendications

1. Stylo à bille avec préservatif, caractérisé par le fait qu'il comporte une partie frontale (1, 2, 34) présentant une pointe d'écriture (1, 26) dans laquelle est disposée une mine (6, 29); par le fait que la partie frontale se prolonge par une douille (3, 4, 25) en une ou plusieurs parties, qui peut être obturée à une extrémité (5) et dans laquelle se trouve au moins un préservatif (K8, K9, K); et par le fait que cette douille est pourvue de moyens (28, 32) facilitant une extraction des préservatifs (K8, K9, K) hors de ladite douille.

2. Stylo à bille selon la revendication 1, caractérisé par le fait que la douille se compose de parties (3, 4) qui sont reliées à la partie frontale et les unes aux autres, de manière libérable, et renferment chacune un préservatif (K8, K9).

3. Stylo à bille selon la revendication 2, caractérisé par le fait que les parties (3, 4) de la douille sont reliées, par vissage, les unes aux autres et à la partie frontale (1, 2).

4. Stylo à bille selon la revendication 1, caractérisé par le fait que la douille renferme un curseur (28) auquel un coulissement peut être imprimé au moyen d'une partie de préhension saillant, vers l'extérieur, à travers une fente longitudinale (32).

## Claims

1. Ball pen with condom, characterized in that it has a head part (1, 2, 34) with a writing tip (1, 26) and a refill (6, 29) positioned thereon, that the head part passes into a terminally (5) sealable single or multiple-part sleeve (3, 4, 25), in which is housed at least one condom (K8, K9, K) and that said sleeve is provided with means (28, 32) facilitating the discharge from the sleeve of the condoms (K8, K9 K).

2. Ball pen according to claim 1, characterized in that the sleeve comprises sleeve parts (3, 4) detachably connected to the head part and to one another and which in each case contain a condom (K8, K9).

3. Ball pen according to claim 2, characterized in that the sleeve parts (3, 4) are screwed to one another and to the head part (1, 2).

4. Ball pen according to claim 1, characterized in that within the sleeve is fitted a slider (28), which is displaceable by means of a grip part projecting outwards through a longitudinal slit (32).

FIG. 1

FIG. 2

FIG. 3